# EUROPEAN PATENT SPECIFICATION

(11) **EP 3 157 433 B1**
(45) Date of publication and mention of the grant of the patent: **23.11.2022**
(21) Application number: 15809658.6
(22) Date of filing: 19.06.2015
(51) Int. Cl.: A61B 5/18, A61B 5/11, A61B 3/113, G06K 9/00, G06V 20/59, G06V 40/18

(54) **MONITORING DROWSINESS**
ÜBERWACHUNG VON BENOMMENHEIT
SURVEILLANCE DE SOMNOLENCE

(30) Priority: 20.06.2014 AU 2014902364
(43) Date of publication of application: 26.04.2017
(73) Proprietor: SDIP HOLDINGS PTY LTD, Victoria 3121 (AU)
(72) Inventor: MORGAN, Trefor, VIC 3056 (AU); COLES, Scott, NSW 2060 (AU); TUCKER, Andrew, VIC 3094 (AU)
(74) Representative: Sandersons
(86) International application number: PCT/AU2015/000359
(87) International publication number: WO 2015/192171

(56) References cited:
- EP-A1- 2 410 501
- WO-A1-2006/092022
- WO-A1-2014/031042
- US-A1- 2008 150 734
- US-B2- 7 791 491
- WIERTS R ET AL: "Measuring Saccade Peak Velocity Using a Low-Frequency Sampling Rate of 50 Hz", IEEE TRANSACTIONS ON BIOMEDICAL ENGINEERING, IEEE SERVICE CENTER, PISCATAWAY, NJ, USA, vol. 55, no. 12, 1 December 2008 (2008-12-01), pages 2840-2842, XP011249417, ISSN: 0018-9294
- CASTIGLIONI P ET AL: "Interpolation technique for extracting features from ECG signals sampled at low sampling rates", COMPUTERS IN CARDIOLOGY 2003 (IEEE CAT. NO.03CH37504) IEEE PISCATAWAY, NJ, USA, 2003, pages 481-484, XP002777284, ISBN: 0-7803-8170-X
- ANDREW D. OUZTS, ANDREW T. DUCHOWSKI: "Comparison of eye movement metrics recorded at different sampling rates", ETRA '12 PROCEEDINGS OF THE SYMPOSIUM ON EYE TRACKING RESEARCH AND APPLICATIONS, 28 March 2012 (2012-03-28), pages 321-324, XP002777285, Santa Clara, Califirnia, USA DOI: 10.1145/2168556.2168626 ISBN: 978-1-4503-1221-9
- S. BOVERIE, A. GIRALT: "Driver vigilance diagnostic based on eyelid movement observation", THE INTERNATIONAL FEDERATION OF AUTOMATIC CONTROL, vol. 41, no. 2, 6 July 2008 (2008-07-06), pages 12831-12836, XP002777286, DOI: 10.3182/20080706-5-KR-1001.02170
- FABIAN FRIEDRICHS ET AL: "Camera-based drowsiness reference for driver state classification under real driving conditions", INTELLIGENT VEHICLES SYMPOSIUM (IV), 2010 IEEE, IEEE, PISCATAWAY, NJ, USA, 21 June 2010 (2010-06-21), pages 101-106, XP031732200, ISBN: 978-1-4244-7866-8

## Description

This invention relates to methods of measuring drowsiness and in particular of using eyelid movement data from any source that has low sampling rates.

### Background to the invention.

USA patents 7071831, 7616125 and 7791491 relate to a method and algorithm for predicting the onset of potentially fatal drowsiness. The method in practice uses spectacle mounted I R sensors to sense eye and eyelid movement and measure amplitude and velocity of these movements for processing in an algorithm that provides a measure that is applicable to a scale of drowsiness. Other sensor systems such as cameras could be used to collect this data but have not been used as their sampling rates are low. The attraction of a camera based system is that spectacle need not be worn to collect the data and is thus less intrusive.

USA patent 7043056 to a camera based method of determining a head pose measurement. USA patent 7460693 to a camera based method of locating a face within an input image including eye locations. USA patent 7653213 to a face tracking system utilising a Kalman filter and deriving a Jacobian. USA patent 8165347 to a face tracking system which includes a determination if glasses are being worn.

There are many patents to eye tracking methods . Some of these are for use in anticipating a screen users requirements as part of a computer or mobile phone system. Other patents are concerned with identification by iris examination and an example is USA patent 8064647.

Some patents are more focussed on analysing eye movements such as USA patent 7809160 for eye tracking without camera calibration. FABIAN FRIEDRICHS ET AL, "Camera-based drowsiness reference for driver state classification under real driving conditions," INTELLIGENT VEHICLES SYMPOSIUM (IV), 2010 IEEE, PISCATAWAY, NJ, USA, 21 June 2010, pages 101-106, ISBN: 978-1-4244-7866-8 discloses a camera-based drowsiness detection.

Camera based systems have been proposed for detecting drowsiness but their ability to provide reliable early predictions in a high percentage of cases is questionable.

It is an object to provide a method and apparatus that enables other sensor inputs of eye movement data.

### Brief description of the invention

To this end the present invention provides a method of determining drowsiness which includes the steps of receiving eye movement data collected by a camera at sampling rate greater than 20 Hz but less than 250 Hz

interpolating the data to provide a data set sampling rates greater than 250Hz for each data point deriving values of amplitude and velocity of eye movement and whether the measures relate to eyelid opening or closing using an algorithm to obtain values of the amplitude to velocity ratios of eyelid opening and closing and using these values in an algorithm for providing a measure of drowsiness.

This invention is predicated on the unexpected discovery that lower sampling rates can provide sufficient data for use in the method of USA patents 7071831, 7616125 and 779149. The system of those patents uses a sample rate of 500Hz to gather amplitude measurements for eyelid movements. In this invention the sampling rates for various type of sensor devices may be 50Hz, 100Hz, 200Hz. Preferably the upper limit for the sample rate of the eye movement data source is 200Hz.

An alternate embodiment of the invention avoids the need to adjust the sample rate of the eye movement data source (such as that obtained from a video camera). Modifications to the methods of collation and calculation of eye movement amplitude, velocity and durations used in the JDS algorithm may be adjusted to account for low sampling rates.

Thus in another embodiment this disclosure provides a method of determining drowsiness which includes the steps of receiving eye movement data collected at sampling rate greater than 20 Hz but less than 250 Hz collating and calculating eye movement amplitude, velocity and duration;
for each data point, deriving values of amplitude and velocity of eye movement and whether the measures relate to eyelid opening or closing;
using an algorithm to obtain values of the amplitude to velocity ratios of eyelid opening and closing and
using these values in an algorithm for providing a measure of drowsiness.

The amplitude to velocity ratio for eyelid opening and closing are used as the main measure of drowsiness onset In the John Drowsiness Scale (JDS). The ratio of the amplitude of to the maximum velocity (AVR) for both closing and opening phases during blinks increases with drowsiness and can be used to predict lapses in vigilance. The AVR for eyelid closure and reopening are different for the same amplitude. Generally eyelids close more quickly than they reopen and the two velocities are only moderately correlated. Sleep deprivation , restriction or other reasons for drowsiness increases AVR for both closing and reopening. Consequently the duration of these movements increase with drowsiness. It has been found that the ratio of opening and closing velocities with their respective amplitudes are a major indicator of drowsiness. The ratio of the amplitude of opening and closing movements relative to the maximum velocity (AVR) of these movements has the dimension of time and is relatively constant with alert subjects but increases progressively with drowsiness and does not require calibration.

The values calculated for the purposes of comparison need to be collated over a predetermined period of time and expressed in an appropriate way. These calculations are preferentially expressed as averages, standard deviations, percentiles or counts. The eyelid parameters measured and the values selected for calculation can be determined by conducting trials and may be any suitable combination of parameters. Preferably the velocity to amplitude ratios are calculated for each detected movement and then expressed as averages and standard deviations over a predetermined interval. Other parameters such as duration of opening, closure and closing may also be collated and included in the final calculated value. Eye movements such as saccades may be used as additional parameters if they are available from the data source. The various parameters are preferably weighted in reaching the final calculation. This final calculation becomes an index of drowsiness with a low value indicating alertness and higher values indicating increasing levels of drowsiness.

Eyelid and eye movement may be monitored using any suitable technology or sensors including video or digital camera technology to identify and measure the appropriate eye movements.

### Detailed description of the invention

A preferred embodiment of the invention will now be described with reference the drawings in which:
Figure 1A is a good quality eyelid aperture data signal derived from video showing a 30 second period of blinks;
Figure 1B is a noisy eyelid aperture data signal derived from video showing a 24 second period of noise between blinks;
Figure 2 illustrates a graph of Johns drowsiness score derived fromvideo based signals over 120 minutes;
Figure 3 illustrates JDS score for 500Hz vs 50Hz; 50Hz to reflect the applicability of JDS for lower sampling rates;
Figure 4 illustrates minimal error of 50Hz signal against the original 500Hz signal.

A data set of eyelid movements obtained using a camera-based system was obtained using a third party program which supplied the data as a CSV output file with time and eye opening (aperture) value for each video sample.

The data was then upscaled to 500 Hz and the amplitude rescaled.

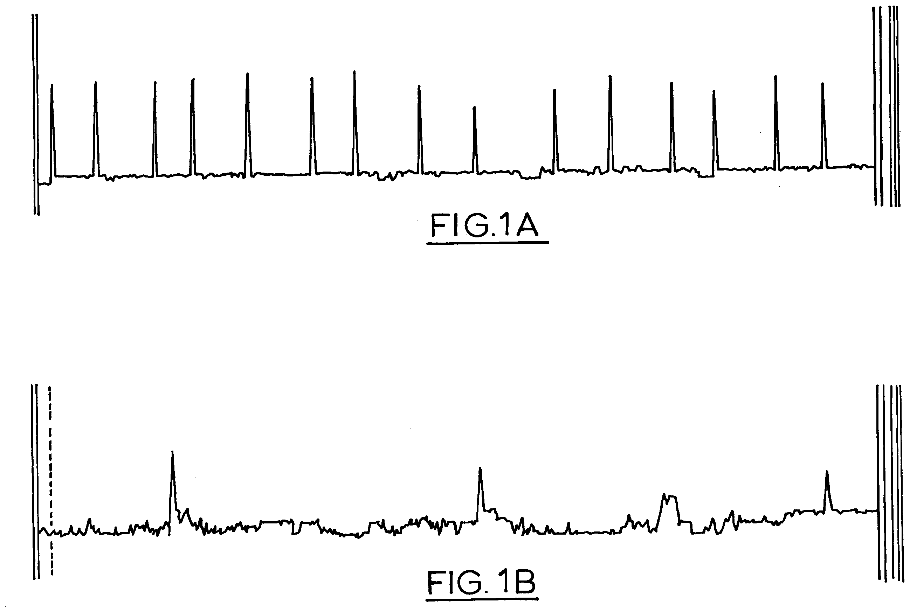

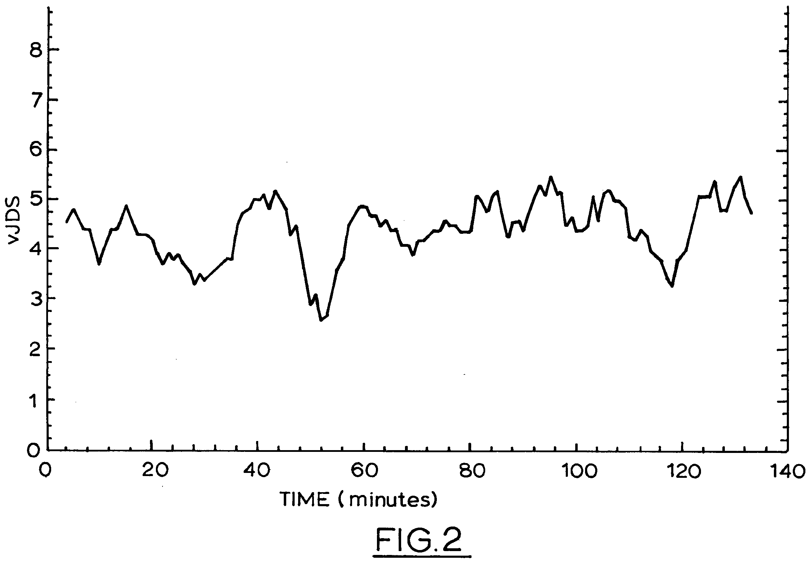

Initial analysis of the data is shown in figures 1A which is a good quality signal and figure 1 B illustrates that blinks can be identified in a noisy signal. After converting and interpolating the video camera output, analysis by the JDS algorithms show in figure 2 a JDS score over a period of two hours.

To investigate the feasibility of using a low sampling rate as in data from a camera, a sample data set with a sampling rate of 500Hz was adjusted to a sample rate of 50Hz. This was then converted back up to a sample rate of 500Hz by interpolating between the data points of the 50Hz sample. Linear interpolation is the preferred method, but other methods of interpolation such as sample and hold, polynomial interpolation or spline interpolation can be used.

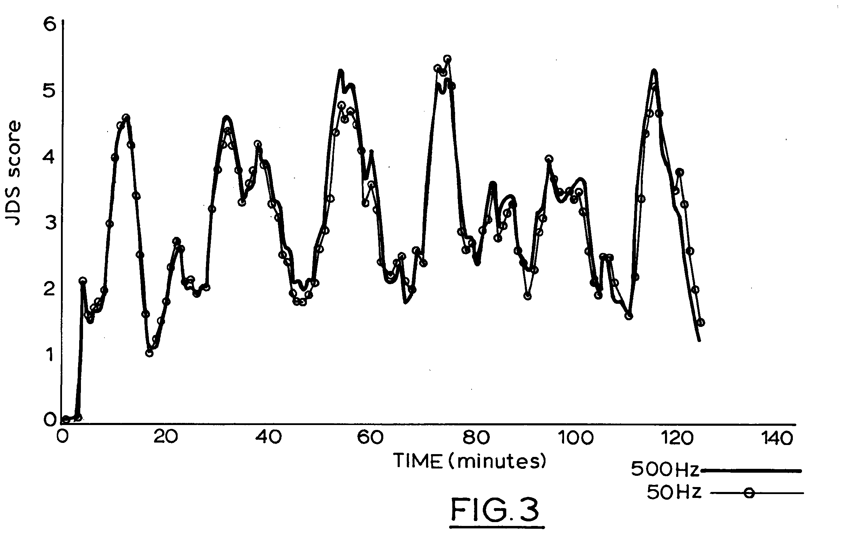

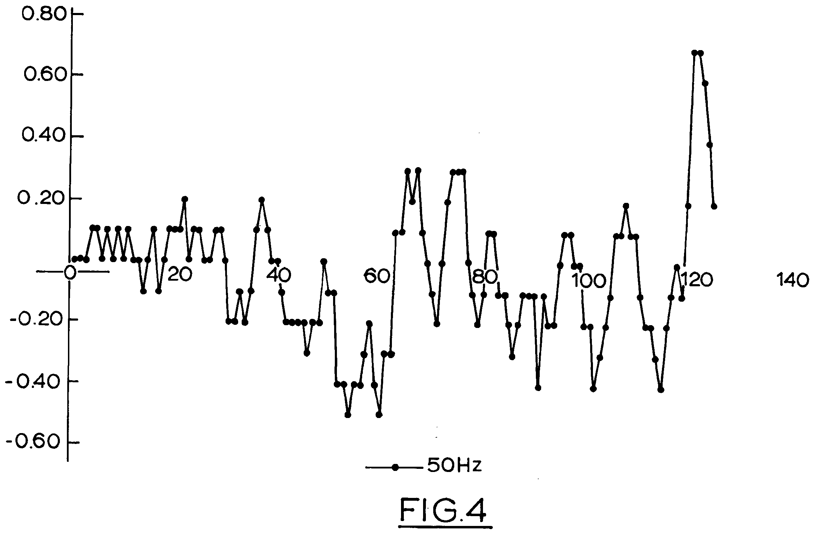

Figure 3 illustrates the difference in the JDS scores over time derived from the original 500Hz data and that derived from the 50Hz data set. Figure 4 illustrates the error of the JDS scores derived from the 50Hz data set against the original 500Hz data set. This demonstrates that the use of a lower sampling frequency provides reliable scores.

An alternate embodiment of the invention is possible without the need to adjust the sample rate of the eye movement data source (such as that obtained from a video camera). Modifications to the methods of collation and calculation of eye movement amplitude, velocity and durations may be adjusted to account for alternate sampling rates.

Those skilled in the art will realize that this invention improves the functionality of the applicants drowsiness algorithm by making it able to accept data collected by any means at lower sampling frequencies.

## Claims

1. A method of determining drowsiness which includes the steps of collecting eye movement data using a camera, wherein the eye movement data is eyelid aperture data and is collected at sampling rate greater than 20 Hz but less than 250 Hz;
interpolating the data to provide a data set sampling rates greater than 250 Hz;
for each data point, deriving values of amplitude and velocity of eye movement and whether the measures relate to eyelid opening or closing;
based on the derived values of amplitude and velocity of eye movement, using an algorithm to obtain values of amplitude to velocity ratios of eyelid opening and closing; and
using these values in an algorithm for providing a measure of drowsiness.

## Patentansprüche

1. Verfahren zum Bestimmen von Schläfrigkeit, das die Schritte einschließt
Sammeln von Augenbewegungsdaten unter Verwendung einer Kamera, wobei die Augenbewegungsdaten Augenlidöffnungsdaten sind und mit einer Abtastrate von mehr als 20 Hz, aber weniger als 250 Hz gesammelt werden;
Interpolieren der Daten, um einem Datensatz Abtastraten von mehr als 250 Hz bereitzustellen;
für jeden Datenpunkt, Ableiten von Werten einer Amplitude und einer Geschwindigkeit einer Augenbewegung und ob sich die Messungen auf ein Öffnen oder ein Schließen der Augenlider beziehen;
basierend auf den abgeleiteten Werten der Amplitude und der Geschwindigkeit der Augenbewegung, Verwenden eines Algorithmus, um Werte von Amplituden-zu-Geschwindigkeits-Verhältnissen des Öffnens und des Schließens des Augenlids zu erhalten; und
Verwenden dieser Werte in einem Algorithmus zum Bereitstellen eines Maßes für Schläfrigkeit.

## Revendications

1. Procédé de détermination de somnolence qui comporte les étapes de collecte de données de mouvement oculaire à l'aide d'une caméra, dans lequel les données de mouvement oculaire sont des données d'ouverture de paupière et sont collectées à un taux d'échantillonnage supérieur à 20 Hz mais inférieur à 250 Hz ;
l'interpolation des données pour fournir un ensemble de données à des taux d'échantillonnage supérieurs à 250 Hz ;
pour chaque point de données, la dérivation de valeurs d'amplitude et de vitesse du mouvement oculaire et la détermination du fait que les mesures sont associées à l'ouverture ou à la fermeture de paupière ;
sur la base des valeurs dérivées d'amplitude et de vitesse de mouvement oculaire, l'utilisation d'un algorithme pour obtenir des valeurs de rapports amplitude/vitesse d'ouverture et de fermeture de paupière ; et
l'utilisation de ces valeurs dans un algorithme pour fournir une mesure de somnolence.
